# EUROPEAN PATENT SPECIFICATION

(11) **EP 2 005 988 B1**
(45) Date of publication and mention of the grant of the patent: **16.03.2016**
(21) Application number: 08252096.6
(22) Date of filing: 18.06.2008
(51) Int. Cl.: A61M 25/06, A61M 25/09

(54) **A medical guide wire**
Medizinischer Führungsdraht
Guide-fil médical

(30) Priority: 22.06.2007 JP 2007164836
(43) Date of publication of application: 24.12.2008
(73) Proprietor: ASAHI INTECC CO., LTD., Nagoya-shi, Aichi-ken (JP)
(72) Inventor: Miyata, Naohiko, Nagoya-shi Aichi-ken (JP); Nagano, Satoshi, Nagoya-shi Aichi-ken (JP); Koike, Tadahiro, Nagoya-shi Aichi-ken (JP)
(74) Representative: Nicholls, Michael John

(56) References cited:
- EP-A- 1 772 167
- WO-A-2005/053529
- US-A1- 2001 021 831
- US-A1- 2002 074 051
- US-A1- 2007 010 762

## Description

The invention relates to a medical guide wire well-suited to a medical usage, and particularly concerns to a medical guide wire used upon inserting a catheter into a blood vessel, urethra and organs, and also used upon leading a retainer to an aneurysm-forming tract area.

In a medical guide wire, a flexibility is required for a distal end of the guide wire not to have a damage on a blood vessel, while at the same time, a sufficient torque-transmissibility is required to transmit a manual operat ion from a proximal side to a distal end of the guide wire.

In order to satisfy the above requirements, a core shaft is prepared, a distal end portion of which is thinned and a rear end of the core shaft is thickened. The core shaft is fixed to a helical spring body with the core shaft inserted into the helical spring body.

The helical spring body is formed by bonding a front helical spring to a basal helical spring, the former of which is made of a single wire. This type of the guide wire is disclosed by Japanese Laid-open Patent Application Nos. 2005-46603, 2006-271955, Japanese Patent Publication No. 4-25024 and Japanese Patent No. 2737650.

Japanese Laid-open Patent Application No. 2005-46603 discloses a guide wire which has a coiled spring provided to enclose a distal end portion of core shaft. A fixing member is provided to concentrically secure the coiled spring to the core shaft, so that the distal end of the guide wire does not partially deform upon steering the guide wire through the somatic cavity.

Japanese Laid-open Patent Application No. 2006-271955 discloses a guide wire in which a core shaft has a projection axially extended in a concentrical relationship with the core shaft. A coiled spring is concentrically provided to enclose a distal end portion of the guide wire, and bonded to the projection, so that the distal end of the guide wire does not partially deform upon inserting the guide wire into the somatic cavity.

Japanese Patent Publication No. 4-25024 discloses a guide wire having a flexible tubular core made of a high torsion metal. A first coiled spring made of a metallic material, and secured to a distal end portion of the tubular core. A second coiled spring made of a radiopaque material, and connected to a distal end of the first coiled spring so as to exhibit a high flexibilty and good torque-transmissibility upon inserting the guide wire into the cardiovascular tract.

Japanese Patent No. 2737650 discloses a coiled connector, one end of which is screwed to a distal helical spring, and the other end of which is screwed to a basal helical spring. This enables manufacturers to connect the distal helical spring to the basal helical spring without sacrificing a good flexibility at a portion in which the distal helical spring is connected to the basal helical spring.

EP 1 772 167 A2 discloses a medical guide wire formed by a helical hollow wire body that is formed into a wire-stranded helical hollow tube by stranding a multitude of wire coil elements. An elongate core is inserted into the wire-stranded helical hollow tube. Both end portions of the elongate core are tapered off.

In the prior art guide wires, when a distal end portion of the guide wire is thinned to decrease its diameter so as to make it more flexible, the thinned distal end portion decreases the torque-transmissibility, thus making it difficult to transmit a basal side operation to the distal end portion of the guide wire.

Therefore, it is an aim of the invention to overcome the above drawbacks so as to provide a medical guide wire which is capable of enhancing a flexibility of a front helical spring while insuring a sufficient torque-transmissibility.

According to the present invention, a medical guide wire is provided to have a core shaft, a distal end side of which is thinned to have a reduced diameter, and a proximal end side of which is thickened to have an increased diameter. A helical spring body has a front helical spring provided at a distal side of the helical spring body and a basal helical spring provided at a rear side of the front helical spring. The core shaft and the helical spring body are fixed together with the distal end side of the core shaft inserted into the helical spring body. The front helical spring has stranded wires formed by a plurality of wires stranded in a helically-wired spring configuration. The basal helical spring is formed by a single wire in a helically-wired spring configuration.

With the front helical spring formed by the stranded wires upon making the guide wire, it is possible to make the guide wire more flexible without thinning the core shaft while insuring a sufficient torque-transmissibility.

According to the other aspect of the present invention, the front helical spring has a stranded wire unit formed by stranding three to nineteen wires in the helically-wired spring configuration.

According to the other aspect of the present invention, the front helical spring has the stranded wire units counted as nine to nineteen. These stranded wire units are further stranded to form the helically-wired multiple spring configuration.

With the helically-wired multiple spring configuration, it is possible to enhance a mechanical strength, compared to the case in which one stranded wire unit is formed in the helically-wired spring configuration.

According to the other aspect of the present invention, the stranded wire units are made of a metallic wire selected from the group consisting of stainless steel wire, Ni-Ti alloyed wire and radiopaque wire, otherwise the stranded wire units are made of at least two kinds of metallic wires selected from the group consisting of stainless steel wire, Ni-Ti alloyed wire and radiopaque wire.

According to the other aspect of the present invention, the front helical spring uses two stranded wire units to form the helically-wired multiple spring configuration, and one of the two stranded wire units is made of a metallic wire selected from the group consisting of stainless steel wire, Ni-Ti alloyed wire and radiopaque wire, and the other hand, the rest of the two stranded wire units is made of at least two kinds of metallic wires selected from the group consisting of stainless steel wire, Ni-Ti alloyed wire and radiopaque wire.

According to the other aspect of the present invention, one of a rear end of the front helical spring and a forward end of the basal helical spring is diametrically reduced integrally to have a diameter-reduced end, so that the diameter-reduced end is inserted into the other of the rear end of the front helical spring and the forward end of the basal helical spring to connect the front helical spring to the basal helical spring.

As a prior art method of bonding the front helical spring to the basal helical spring, the front helical spring is meshed with the basal helical spring to be screwed to the basal helical spring by means of soldering. Otherwise, a helical connector is separately prepared, one end of which is meshed with the front helical spring to be screwed to the front helical spring, and other end of which is meshed with the basal helical spring to be screwed to the basal helical spring.

Both of the connecting methods, however, require complicated procedures in which the front helical spring, the basal helical spring and the helical connector are meshed each other to be screwed, thus rendering the assemble procedures time-consuming.

Contrary to the prior art methods, according to the invention, it suffices to insert the diameter-reduced end of the front helical spring or the basal helical spring upon bonding the former to the latter, thus making it possible to render the assemble procedures simple and quick.

With the diameter-reduced end integrally formed on the front helical spring or the basal helical spring, it is possible to make whole the assemble simple without increasing the number of the necessary component parts.

According to the other aspect of the present invention, a helical connector is provided which has an outer diameter slightly smaller than inner diameters of both the rear end of the front helical spring and the forward end of the basal helical spring. A front end portion of the helical connector is inserted into the rear end of the front helical spring, and a rear end portion of the helical connector is inserted into the forward end of the basal helical spring.

The helical connector makes it simple to insert the helical connector to the front helical spring and the basal helical spring upon bonding the former to the latter.

The invention will now be described by way of non-limiting examples with reference to the accompanying drawings, in which:
Fig. 1 is a longitudinal cross sectional view a medical guide wire according to a first embodiment of the invention but partly sectioned;
Fig. 2 is an enlarged longitudinal cross sectional view designated by a circle E of Fig. 1;
Fig. 3 is an enlarged longitudinal cross sectional view of a basal helical spring;
Fig. 4 is a longitudinal cross sectional view of a main portion of the medical guide wire according to a second embodiment of the invention; and
Fig. 5 is a longitudinal cross sectional view of a main portion of the medical guide wire according to a third embodiment of the invention.

In the following description of the depicted embodiments, the same reference numerals are used for features of the same type.

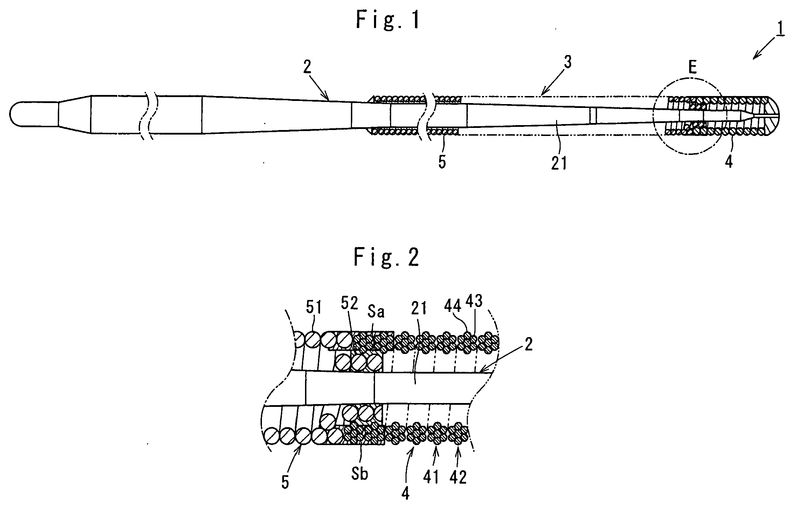

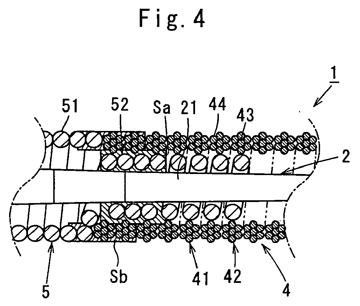

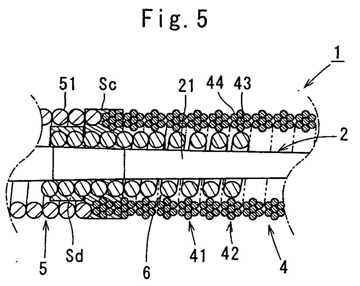

Referring to Figs. 1 through 3, described is a medical guide wire 1 according to a first embodiment of the invention. In Figs. 1 through 3, a right hand side stands a distal end side and a left hand side represents a proximal or rear end side. The same is true with second and third embodiments in Figs. 4 and 5.

The medical guide wire 1 (shortened as "guide wire 1" hereinafter) has a core shaft 2 and a helical spring body 3. The helical spring body 3 is secured to the core shaft 2 with a distal end portion 21 of the core shaft 2 inserted into the helical spring body 3.

The core shaft 2 is made of stainless steel, Ti-Ni based alloy or the equivalents. The core shaft 2 has a distal end portion thinned to have a decreased diameter and having a rear end portion thickened to have an increased diameter.

The helical spring body 3 has a front helical spring 4 and a basal helical spring 5 provided at a rear end of the front helical spring 4.

The front helical spring 4 is formed by two stranded wire units 41, 42 which are arranged consecutively in a helically-wired multiple spring configuration. Each of the stranded wire units 41, 42 has seven wire elements consisting of one core wire 43 and six side wires 44. The six side wires 44 are stranded around the core wire 43.

The core wire 43 is made of stainless steel, and the side wires 44 are made of radiopaque material (e.g., Pt-Ni based alloy).

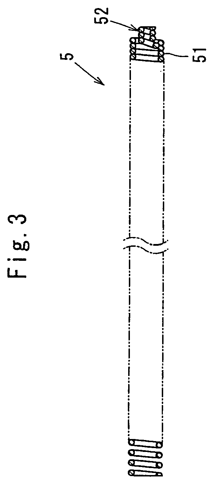

The basal helical spring 5 is formed by a single wire 51 in a helically-wired spring configuration. A diameter of the forward end of the basal helical spring 5 is smaller than that of the rear portion of the basal helical spring 5 which integrally forms a diameter-reduced end 52 as shown in Fig. 3.

An outer diameter of the diameter-reduced end 52 is slightly smaller than an inner diameter of a rear end of the front helical spring 4. The basal helical spring 5 inserts its diameter-reduced end 52 into the rear end of the front helical spring 4 with a permissible clearance presented therebetween. The diameter-reduced end 52 is soldered to the core shaft 2, while at the same time, the diameter-reduced end 52, the front helical spring 4 and the basal helical spring 5 are fittingly bonded by means of soldering Sa, Sb. It is to be noted that the basal helical spring 5 is made of a high rigidity material such as, for example, stainless steel or cobalt-based alloy. On the outer surface of the helical spring body 3, a hydrophilic layer is coated, although not shown.

By way of illustration, the helical spring body 3 is 200 mm long, the front helical spring 4 is 30 mm long, and the basal helical spring 5 is 170 mm long. The core wire 43 and the side wire 44 of the stranded wire units 41, 42 have an outer diameter of 0. 025 mm. An outer diameter of the front helical spring 4 measures 0.33 mm, and an inner diameter of the front helical spring 4 measures 0.18 mm.

An outer diameter of the single wire 51 measures 0.05 mm. An outer diameter of the basal helical spring 5 measures 0.33 mm, and an inner diameter of the basal helical spring 5 measures 0.23 mm. An outer diameter of the diameter-reduced end 52 measures 0.17 mm with its length as 0.5 mm.

With the front helical spring 4 formed by the stranded wire units 41, 42, it is possible to make the guide wire more flexible, while at the same time, insuring a sufficient torque-transmissibility, compared to the case in which a front helical spring is formed by a single wire to be diametrically identical to the front helical spring 4. With the flexibility insured by the front helical spring 4, it is possible to reduce a risk of having a damage on the blood vessel, so as to safely implement the vascular treatment therapeutically.

With the front helical spring 4 formed by the stranded wire units 41, 42, it is possible to increase a rupture-resistance against a torsional deformation, compared to the case in which the front helical spring is formed by the single wire. This is also preferable to safely implement the vascular treatment.

With the diameter-reduced end 52 inserted into the rear end of the front helical spring 4 upon bonding the basal helical spring 5 to the front helical spring 4, needed is such a simple and quick procedure as to insert the diameter-reduced end 52 into the front helical spring 4.

With the diameter-reduced end 52 soldered to the front helical spring 4, it is possible to insure a sufficient bonding strength between the diameter-reduced end 52 and the front helical spring 4 without resorting to the case in which the two helical springs are meshed and screwed to be bonded together by means of the soldering procedure.

With the diameter-reduced end 52 integrally formed with the basal helical spring 5, it is possible to assemble the guide wire 1 without increasing the number of the necessary component parts.

Even if an excessive tensile force disconnectedly breaks the distal end portion 21 of the core shaft 2, the tensile force deforms the front helical spring 4 to diametrically shrink around a remained portion of the core shaft 2 so as to develop a considerable amount of frictional resistance between an inner surface of the front helical spring 4 and an outer surface of the core shaft 2. The frictional resistance counters the tensile force working at a bonded portion between the front helical spring 4 and the basal helical spring 5. This helps to avoid the disconnection betweeen the front helical spring 4 and the basal helical spring 5 so as to contribute to safely implementing the vascular treatment with the guide wire 1.

Fig. 4 shows a second embodiment of the invention in which the diameter-reduced end 52 is expanded along the core shaft 2 toward the distal side of the guide wire 1. The diameter-reduced end 52 is expanded in a tensile direction to broaden the helical pitch until the diameter-reduced end 52 reaches 10 mm in length from the original length (6 mm). The broadened helical pitch makes the diameter-reduced end 52 more flexible without excessively increasing the rigidity at the bonded portion between front helical spring 4 and basal helical spring 5, while at the same time, insuring a good restitutive capability.

Fig. 5 shows a third embodiment of the invention in which a helical connector 6 is discretely provided. The guide wire 1 has the core shaft 2, the helical spring body 3 and the helical connector 6. The helical connector 6 has an outer diameter slightly smaller than an inner diameter of the rear end of the front helical spring 4 and an inner diameter of the forward end of the basal helical spring 5.

A front end of the helical connector 6 is fittingly inserted into the rear end of the front helical spring 4, and a rear end of the helical connector 6 inserted into the forward end of the basal helical spring 5 so as to connect the front helical spring 4 to the basal helical spring 5 via the helical connector 6. The front helical spring 4, the basal helical spring 5 and the helical connector 6 are bonded together by means of solders Sc, Sd. The helical connector 6 is also bonded to the core shaft 2 by means of the solders Sc, Sd.

Upon connecting the front helical spring 4 to the basal helical spring 5, needed are such simple and quick procedures as to insert the helical connector 6 to the front helical spring 4 and the basal helical spring 5.

The helical connector 6 makes the complicated prior procedures unnecessary in which one helical spring is meshed with another helical spring to screw the former to the latter.

### MODIFICATION FORMS

(A) The front helical spring 4 is formed by stranding the six side wires 44 around one core wire 43, however, the number of the wire elements for the front helical spring 4 is not confined only to seven.
   The number of the wire elements for the stranded wire units 41, 42 may be within the range of three to nineteen. The number of the wire elements for the stranded wire unit 41 may differ from the number of the wire elements for the stranded wire unit 42.
(B) Instead of the two stranded wire units 41, 42, only one stranded wire unit may be used, otherwise more than three stranded wire units may be employed, although the two stranded wire units 41, 42 are preferable to preserve the flexibility and mechanical strength.
(C) Instead of the core wire 43 made of the stainless steel and the side wires 44 made of the radiopaque wire, the core wire 43 and the side wires 44 may be made from the same material such as the stainless steel, Ni-Ti based alloy or radiopaque material.
(D) In lieu of the core wire 43 made of the stainless steel and the side wire 44 made of the radiopaque material, the combination of core wire 43 and the side wire 44 may be made from at least two different kinds of material selected from the group consisting of the stainless steel, Ni-Ti based alloy and radiopaque material.
(E) The stranded wire unit 41 may be made from one metal selected from the group consisting of the stainless steel, Ni-Ti based alloy and radiopaque material, and the stranded wire unit 42 may be made from at least two different kinds of material selected from the group consisting of the stainless steel, Ni-Ti based alloy and radiopaque material.
(F) In place of the diameter-reduced end 52 integrally formed on the forward end of the basal helical spring 5, the diameter-reduced end 52 may be formed on the rear end of the front helical spring 4 to be inserted into the forward end of the basal helical spring 5 to connect the front helical spring 4 to the basal helical spring 5.
(G) Otherwise, the front helical spring 4 may be meshed with the basal helical spring 5 to be screwed to the basal helical spring 5, and the former is fittingly bonded to the latter by means of soldering procedure.
(H) Alternatively, a discrete helical connector may be provided, one end of which is screwed to respective one end of the front helical spring, and the other end of which is screwed to one end of the basal helical spring upon connecting the front helical spring to the basal helical spring.

## Claims

1. A medical guide wire (1) having:
a core shaft (2), a distal end side (21) of which is thinned to have a reduced diameter; and
a helical spring body (3) having a front helical spring (4) provided at a distal side of said helical spring body (3) and a basal helical spring (5) provided at a rear side of said front helical spring (4);
said core shaft (2) and said helical spring body (3) being fixed together with said distal end side of said core shaft (2) inserted into said helical spring body (3);
**characterised in that**,
said front helical spring (4) having stranded wires formed by a plurality of wires stranded in a helically-wired spring configuration;
a proximal end side of the core shaft (2) is thickened to have an increased diameter; and
said basal helical spring (5) is formed by a single wire in a helically-wired spring configuration.

2. The medical guide wire (1) according to claim 1, wherein said front helical spring (4) having a stranded wire unit (41, 42) formed by stranding three to nineteen wires in said helically-wired spring configuration.

3. The medical guide wire (1) according to claim 1 or 2, wherein said front helical spring (4) has a plurality of said stranded wire units (41, 42) further stranded to be formed in helically-wired multiple spring configuration.

4. The medical guide wire (1) according to claim 1 or 2, wherein said stranded wire units (41, 42) are made of a metallic wire selected from the group consisting of stainless steel wire, Ni-Ti alloyed wire and radiopaque wire, otherwise said stranded wire units (41, 42) are made of at least two kinds of metallic wires selected from the group consisting of stainless steel wire, Ni-Ti alloyed wire and radiopaque wire.

5. The medical guide wire (1) according to claim 3, wherein said front helical spring (4) of said helical spring body (3) uses two stranded wire units (41, 42) to form said helically-wired multiple spring configuration, and one of said two stranded wire units (41, 42) is made of a metallic wire selected from the group consisting of stainless steel wire, Ni-Ti alloyed wire and radiopaque wire, and the rest of said two stranded wire units (41, 42) is made of at least two kinds of metallic wires selected from the group consisting of stainless steel wire, Ni-Ti alloyed wire and radiopaque wire.

6. The medical guide wire (1) according to any one of the preceding claims, wherein one of a rear end of said front helical spring (4) and a forward end of said basal helical spring (5) is diametrically reduced integrally to have a diameter-reduced end (52), so that said diameter-reduced end is inserted into the other of said rear end of said front helical spring (4) and said forward end of said basal helical spring (5).

7. The medical guide wire (1) according to any one of claims 1 to 5, wherein a helical connector (6) is provided which has an outer diameter slightly smaller than inner diameters of both said rear end of said front helical spring (4) and said forward end of said basal helical spring (5), and a front end portion of said helical connector (6) is inserted into said rear end of said front helical spring (4), and a rear end portion of said helical connector (6) is inserted into said forward end of said basal helical spring (5).

## Patentansprüche

1. Medizinisches Führungskabel (1), umfassend:
einen Kernschaft (2), von dem eine distale Endseite (21) verdünnt ist, um einen verkleinerten Durchmesser anzunehmen; und
einen Spiralfederkörper (3) mit einer vorderen Spiralfeder (4) an einer distalen Seite des Spiralfederkörpers (3) und einer basalen Spiralfeder (5) an einer Rück - seite der vorderen Spiralfeder (4); wobei der Kernschaft (2) und der Spiralfeder - körper (3) aneinander befestigt sind, indem die distale Endseite des Kernschafts (2) in den Spiralfederkörper (3) eingesetzt ist,
**dadurch gekennzeichnet,**
**dass** die vordere Spiralfeder (4) verseilte Drähte aufweist, gebildet durch mehrere Drähte, die in einer spiralförmig verdrahteten Federkonfiguration verseilt sind;
eine proximale Endseite des Kernschafts (2) zu einem vergrößertem Durch - messer verdickt ist; und
die basale Spiralfeder (5) durch einen einzelnen Draht in einer spiralförmig verdrahteten Federkonfiguration gebildet ist.

2. Medizinisches Führungskabel (1) nach Anspruch 1, bei dem die vordere Spi - ralfeder (4) mit einer verseilten Drahteinheit (41, 42) ausgestattet ist, gebildet durch Verseilen von drei bis neunzehn Drähten in der spiralförmig verdrahteten Federkonfiguration.

3. Medizinisches Führungskabel (1) nach Anspruch 1 oder 2, bei dem die vorde - re Spiralfeder (4) eine Mehrzahl der verseilten Drahteinheiten (41, 42) aufweist, die weiterhin verseilt sind, um eine spiralförmig verdrahtete Mehrfach-Federkonfi - guration zu bilden.

4. Medizinisches Führungskabel (1) nach Anspruch 1 oder 2, bei dem die ver - seilten Drahteinheiten (41, 42) aus einem Metalldraht gebildet sind, ausgewählt aus der Gruppe Edelstahldraht, Ni-Ti-Legierungsdraht und röntgendichter Draht, im Übrigen die verseilten Drahteinheiten (41, 42) aus mindestens zwei Arten von Metalldrähten gebildet sind, ausgewählt aus der Gruppe Edelstahldraht, Ni-Ti-Legierungsdraht und röntgendichter Draht.

5. Medizinisches Führungskabel (1) nach Anspruch 3, bei dem die vordere Spi - ralfeder (4) des Spiralfederkörpers (3) zwei verseilte Drahteinheiten (41, 42) zum Bilden der spiralförmig verdrahteten Mehrfach-Federkonfiguration verwendet, und eine der beiden verseilten Drahteinheiten (41, 42) aus einem Metalldraht gefertigt ist, ausgewählt aus der Gruppe Edelstahldraht, Ni-Ti-Legierungsdraht und rönt - gendichter Draht, während der Rest der zwei verseilten Drahteinheiten (41, 42) aus mindestens zwei Arten von Metalldrähten gebildet ist, ausgewählt aus der Gruppe Edelstahldraht, Ni-Ti-Legierungsdraht und röntgendichter Draht.

6. Medizinisches Führungskabel (1) nach einem der vorhergehenden Ansprü - chen, bei dem eine von der hinteren und der vorderen Spiralfeder (4) und ein vor - deres Ende der basalen Spiralfeder (5) im Durchmesser integral verringert ist, um ein im Durchmesser verkleinertes Ende (42) aufzuweisen, so dass das im Durch - messer verkleinerte Ende in die andere von der hinteren und der vorderen Spiral - feder (4) und das vordere Ende der basalen Spiralfeder (5) eingesetzt ist.

7. Medizinisches Führungskabel (1) nach einem der Ansprüche 1 bis 5, bei dem ein Spiralverbinder (6) vorgesehen ist, der einen Außendurchmesser etwas klei - ner als die Innendurchmesser sowohl der hinteren als auch der vorderen Spiralfe - der (4) und des vorderen Endes der basalen Spiralfeder (5) aufweist, und ein vor - derer Endbereich des Spiralverbinders (6) in das hintere Ende der vorderen Spi - ralfeder (4) eingesetzt ist, und ein hinterer Endbereich des Spiralverbinders (6) in das vordere Ende der basalen Spiralfeder (5) eingesetzt ist.

## Revendications

1. Fil de guidage médical (1), comportant :
un arbre de coeur (2), dont un côté d'extrémité distale (21) est aminci de façon à avoir un diamètre réduit ; et
un corps de ressort hélicoïdal (3) comportant un ressort hélicoïdal avant (4) disposé à un côté distal dudit corps de ressort hélicoïdal (3) et un ressort hélicoïdal de base (5) disposé à un côté arrière dudit ressort hélicoïdal avant (4) ;
ledit arbre de coeur (2) et ledit corps de ressort hélicoïdal (3) étant fixés l'un à l'autre avec le côté d'extrémité distale dudit arbre de coeur (2) inséré dans ledit corps de ressort hélicoïdal (3) ;
**caractérisé en ce que** :
ledit ressort hélicoïdal avant (4) comporte des fils torsadés constitués par une pluralité de fils torsadés sous une configuration de ressort à fils hélicoïdaux ;
un côté d'extrémité proximale de l'arbre de coeur (2) est épaissi de façon à avoir un diamètre accru ; et
ledit ressort hélicoïdal de base (5) est constitué par un fil unique sous une configuration de ressort à fil hélicoïdal.

2. Fil de guidage médical (1) selon la revendication 1, dans lequel ledit ressort hélicoïdal avant (4) comporte une unité de fils torsadés (41, 42) constituée par le fait de torsader de trois à dix-neuf fils sous ladite configuration de ressort à fils hélicoïdaux.

3. Fil de guidage médical (1) selon la revendication 1 ou 2, dans lequel ledit ressort hélicoïdal avant (4) comporte une pluralité desdites unités de fils torsadés (41, 42), torsadées de plus de façon à être formées sous une configuration de ressort multiple à fils hélicoïdaux.

4. Fil de guidage médical (1) selon la revendication 1 ou 2, dans lequel lesdites unités de fils torsadés (41, 42) sont constituées par un fil métallique sélectionné parmi le groupe comprenant un fil en acier inoxydable, un fil en alliage Ni-Ti et un fil radio-opaque, ou, d'une autre façon, lesdites unités de fils torsadés (41, 42) sont constituées par au moins deux types de fils métalliques sélectionnés parmi le groupe comprenant un fil en acier inoxydable, un fil en alliage Ni-Ti et un fil radio-opaque.

5. Fil de guidage médical (1) selon la revendication 3, dans lequel ledit ressort hélicoïdal avant (4) dudit corps de ressort hélicoïdal (3) utilise deux unités de fils torsadés (41, 42) pour former ladite configuration de ressort multiple à fils hélicoïdaux, et l'une desdites deux unités de fils torsadés (41, 42) est constituée par un fil métallique sélectionné parmi le groupe comprenant un fil en acier inoxydable, un fil en alliage Ni-Ti et un fil radio-opaque, et le reste desdites deux unités de fils torsadés (41, 42) est constitué par au moins deux types de fils métalliques sélectionnés parmi le groupe comprenant un fil en acier inoxydable, un fil en alliage Ni-Ti et un fil radio-opaque.

6. Fil de guidage médical (1) selon l'une quelconque des revendications précédentes, dans lequel l'une d'une extrémité arrière dudit ressort hélicoïdal avant (4) et d'une extrémité avant dudit ressort hélicoïdal de base (5) est diamétralement réduite de façon intégrée de manière à comporter une extrémité de diamètre réduit (52), de telle sorte que ladite extrémité de diamètre réduit soit insérée dans l'autre de ladite extrémité arrière dudit ressort hélicoïdal avant (4) et de ladite extrémité avant dudit ressort hélicoïdal de base (5).

7. Fil de guidage médical (1) selon l'une quelconque des revendications 1 à 5, dans lequel un connecteur hélicoïdal (6) est prévu, lequel a un diamètre extérieur légèrement inférieur aux diamètres intérieurs, tout à la fois, de ladite extrémité arrière dudit ressort hélicoïdal avant (4) et de ladite extrémité avant dudit ressort hélicoïdal de base (5), et une partie d'extrémité avant dudit connecteur hélicoïdal (5) est insérée dans ladite extrémité arrière dudit ressort hélicoïdal avant (4), et une partie d'extrémité arrière dudit connecteur hélicoïdal (6) étant insérée dans ladite extrémité avant dudit ressort hélicoïdal de base (5).
